Europäisches Patentamt

European Patent Office

Office européen des brevets

(11) Publication number: **0 132 399**
**A1**

## (12) EUROPEAN PATENT APPLICATION

(21) Application number: **84304969.3**

(22) Date of filing: **20.07.84**

(51) Int. Cl.⁴: **G 01 N 33/52,** G 01 N 33/72, G 01 N 33/96

(30) Priority: **21.07.83 US 515866**

(43) Date of publication of application: **30.01.85 Bulletin 85/5**

(84) Designated Contracting States: **AT CH DE FR GB IT LI NL**

(71) Applicant: **Corning Glass Works, Houghton Park, Corning New York 14831 (US)**

(72) Inventor: **Campbell, Mitchell Douglas, R.D. 1, Box 542 Old Littleton Road, Harvard, MA (US)**
Inventor: **Uretsky, Laura, 9-1 Shadowbrook Lane, Milford, MA 01757 (US)**
Inventor: **Whitney, Nathaniel Ralph, 101 Brook Street, Franklin, MA (US)**
Inventor: **Wilson, Joan Brush, 7 Rainbow Pond Drive, Walpole, MA (US)**

(74) Representative: **Smith, Sydney et al, Elkington and Fife High Holborn House 52/54 High Holborn, London WC1V 6SH (GB)**

(54) Cooximetry quality control reagents.

(57) A cooximetry quality control composition characterised in that it is free from blood-derived components and comprises a solution of one or more dyes, which solution mimics the spectral response of whole blood at a plurality of wavelengths in the visible region of the spectrum is disclosed.

The present invention provides advantage over the prior art.

-1-

"COOXIMETRY QUALITY CONTROL REAGENTS"

This invention relates to cooximetry quality control reagents; more particularly, it relates to cooximetry quality control reagents which are free from blood-derived components.

Blood is a complex medium containing both cellular and non-cellular components. The principal protein of the cellular components is hemoglobin which transports oxygen from the lungs to tissues and carbon dioxide from tissues to the lungs. Thus, the analysis of a patient's blood to determine the total amount of hemoglobin (THb), the portion oxygenated (oxyhemoglobin, $O_2Hb$), the portion oxidized (methemoglobin or hemiglobin, HiHb), the portion complexed with carbon monoxide (carboxyhemoglobin, COHb), and the remaining portion (reduced hemoglobin, HHb) is useful to a physician in evaluating the respiratory status of the patient.

Such analysis is typically carried out spectrophotometrically on a sample of blood in which the cells have been lysed. In hemoglobinometry, the blood sample, after lysis of the cells, is diluted and reacted chemically with, for example, a solution containing an oxidizing agent and cyanide ion. The procedure is used to measure only THb and ordinarily involves a single measurement at 540 nm. See, e.g., N. W. Tietz, Editor, "Fundamentals of Clinical Chemistry", W.B. Saunders Company, New York, 1976.

"Oximetry" is the term which is generally used to mean the determination of more than one hemoglobin species, such as THb and $O_2Hb$, by means of measurements at two different wavelengths in the visible region.

"Cooximetry" generally refers to the process, often automated, in which a plurality of wavelengths is used to quantitate several hemoglobin species, including COHb, in the same sample. The number of wavelengths required is equal to or greater than the number of species to be determined. The specific wavelengths employed depend, at least in part, on the hemoglobin species to be determined, the spectral response curves thereof and the quality of the filters or diffraction gratings used to isolate light having the selected specific wavelengths. For example, one wavelength may be chosen where two species being determined have the largest difference in spectral response and another may be chosen where the same two species have an identical response (the isobestic point). See Tietz, *supra*.

In order to ensure accurate results, a cooximeter requires a clearly defined quality control program. Such a program typically includes the analysis of samples having known concentrations of the various hemoglobin species. For example, Thb, may be determined by hemoglobinometry on a blood sample and the result compared with that obtained by cooximetry. The $O_2Hb$ value then may be set at 100 percent or 0 percent of the THb value by tonometering the sample with oxygen or nitrogen (or by treating the sample with a reducing agent), respectively. Measurement of the tonometered sample by cooximetry then provides an assessment of instrument accuracy for $O_2Hb$, but only at the extremes of the range of possible values. These extremes, however, are not necessarily near the physiological range found in patient samples.

The accuracy of COHb measurements may be assessed by measuring a sample having a known THb concentration which has been tonometered with carbon monoxide to give 100 percent COHb. Alternatively, a gas chromatographic method may be used. The accuracy of HiHb determinations is typically assessed by a spectrophotometric method which depends on the difference in absorbance at 640 nm between HiHb and other hemoglobin species.

The presence of residual COHb or HiHb in a blood sample will interfere with the determination of 100 percent $O_2$Hb or HHb. The extent of this interference is typically a function of the concentration of the interferent and the wavelength chosen for the measurements. Moreover, the preparation of fresh blood samples with known values for the various hemoglobin species is not feasible on a routine basis for the quality control of cooximetry measurements. Such disadvantages dictate a need for readily available cooximetry quality control reagents which may be used directly without further processing.

Although such a need has been recognized, commercially available cooximetry quality control reagents suffer several disadvantages. Such reagents are typically based on red blood cells or hemoglobin solutions. In either case, such reagents have a limited shelf life, even when refrigerated. For example, one set of such reagents contains fixed human red blood cells in a buffer. The reagents must be refrigerated and have a shelf life of two to three months. Different reagents are supplied for three levels of THb and various hemoglobin species, which levels are clinically relevant. A second commercially available set of cooximetry quality control reagents contains human hemoglobin and has a self life of about eight months. Values are given only for THb. A third set of such reagents contains bovine hemoglobin which has been 98 percent converted to COHb. Values are given for $O_2$Hb and HiHb, but are close to zero. This third set of reagents is relatively stable, but does not have hemoglobin species values which are close to the ranges normally found in patients' blood samples.

In addition to the above commercially available quality control reagents, several prior art references are known which describe hemoglobinometry or oximetry quality control reagents.

U.S. Patent No. 3,705,110 relates to a calibrating fluid for automated instruments for blood cell counting and hemoglobin determination. Such fluid comprises an admixture of nigrosin dye and a carbon black dispersion in dilute aqueous solution which exhibits absorbance linearity in the visible and ultra-violet range, which solution had dispersed therein synthetic latex particles having a particle size of from 5 to 15 microns (um) and in an amount of from 10,000 to 22,000 particles per cubic millimetre. The admixture of nigrosin dye and carbon black dispersion is further described in U.S. Patent No. 3,729,427. It should be noted that the nigrosin dye-carbon black dispersion admixture provides a grey solution which exhibits absorbance linearity in the visible and ultra-violet range.

The use of dyes in other calibration or quality control reagents is, of course, well known. For example, U.S. Patent No. 4,001,142 describes the use of a dye in controls for confirming the calibration and performance characteristics of blood gas instrumentation. The dye, however, serves only to distinguish one control reagent from another and to permit the visual detection of air bubbles in the electrode chamber of the blood gas analyzer. This use of a dye is exemplified in at least one commercially available set of blood gas quality control reagents ("CONFIRM", Corning Medical and Scientific, Corning Glass Works, Medfield, Massachusetts, U.S.A.).

U.S. Patent No. 4,126,575 describes a blood control standard which is designed to verify the analysis of an oximeter instrument. Such standard comprises a sealed receptacle containing specially treated red blood cells and a gaseous head space which is at least equal to the volume of the treated blood cells. The special treatment comprises separating the red blood cells from other blood components, washing the separated red blood cells, treating the washed cells with an aldehyde, washing the treated cells and saturating a portion of the washed, treated cells with carbon monoxide. The blood control standard contains a mixture of the specially treated cells with untreated cells. The gaseous head space is filled with a gas comprising 0-15 percent carbon dioxide, 0-25 oxygen and the remainder nitrogen or other inert gas.

Also, reference may be made to the "Proposal for a Certified Standard for Use In Hemoglobinometry", the second and final report prepared by the Division of Medical Sciences, U.S. National Academy of Sciences-National Research Council, R.K. Cannan, Chairman. The report, which was published in Amer. J. Clin. Path., 30, 211-215 (1958), presents the final recommendations of an NAS-NRC Ad Hoc Panel on the establishment of a hemoglobin standard. In selecting cyanmethemoglobin as the standard, the Ad Hoc Panel considered non-protein materials as standards, such as colored glasses and solutions of pigments so prepared as to approximate the absorption by cyanmethemoglobin of the spectral transmission of the filters, prisms or gratings employed in photometers and spectrophotometers. Significantly, such non-protein materials were rejected because (1) no suitable mixture of pigments suggested itself; (2) it would be necessary to provide a series of glass standards to match the sizes and shapes of commonly used cuvettes; and (3) for some instruments, the glasses also would have to match the lens effects of the round cuvettes and the contents thereof.

It therefore is an object of the present invention to provide a cooximetry quality control composition which is free from blood-derived components.

It is another object of the present invention to provide a cooximetry quality control composition which contains only non-protein materials.

A further object of the present invention is to provide a cooximetry quality control composition which possesses long-term stability without refrigeration.

Accordingly, the present invention provides a cooximetry quality control composition which is free from blood-derived components and which comprises a solution of one or more dyes, which solution mimics the spectral response of whole blood at a plurality of wavelengths in the visible region. In preferred embodiments, the solution is substantially aqueous and contains an adjuvant and/or viscosity adjuster.

More particularly, the present invention provides a set of solutions of one or more dyes. The spectral responses or absorbances of these solutions at a plurality of wavelengths in the visible region are similar to the spectral response of fresh human blood. Consequently, when such solutions are treated as samples on a cooximater or spectrophotometer, results are obtained which give known values for the several hemoglobin species in the ranges of interest. The absolute concentrations and the ratios of the various species vary among the solutions, so that the solutions represent low, normal and high total hemoglobin samples. As a set, the solutions are useful for checking the linearity of the measuring channels. Such a three-point check is more desirable than a single-point check since it assesses both accuracy and sensitivity.

The present invention is independent of the cooximeter or spectrophotometer employed to make the necessary measurements of absorbance at a plurality of wavelengths in the visible region. Because different instruments may use different wavelengths and/or different calculation algorithms, a set of solutions prepared for use on a given instrument may not give the expected results for the various hemoglobin species on a different instrument. Based on the present teachings, however, those skilled in the art will appreciate how to prepare a set of solutions for given instruments. The spectral response curves of blood and dyes are known or readily determined. Accordingly, different dye solutions may be formulated by those skilled in the art which will provide the appropriate values for given instrument, provided that the wavelengths used in such instrument are known. The same considerations apply to the preparation of dye solutions for use in the quantity control of measurements involving non-human blood samples, e.g., as in veterinary medicine. Although some experimentation may be necessary to optimize the concentrations of the dyes and other components present in the solution, undue experimentation will not be required.

Consequently, the present invention will be further described with illustrative reference to a specific instrument, the Corning Model 2500 Cooixmeter (Corning Medical and Scientific, Corning Glass Works, Medfield, Massachusetts, U.S.A.). This instrument uses seven discrete wavelengths: 521 nm, 534.5 nm, 547 nm, 585 nm, 595 nm, 627 nm and 660 nm. Using the measured absorbance value of the sample and appropriate extinction coefficient at each wavelength, the instrument calculates the concentrations, in g/dl, of HHb, $O_2Hb$, COHb and HiHb. The instrument sums all four concentrations to give a concentration for THb and then recalculates each measured species as a percentage of THb.

In general, the solvent or solvent mixture used to prepare the solution of one or more dyes is not critical, provided that the dyes and other components are sufficiently soluble therein. Obviously, such solvent or solvent mixture must not adversely affect dye or other component stability to a significant degree.

Typical solvents include water, alcohols, esters, ketones and other organic solvents. Examples of typical solvents include water, methanol, ethanol, propanol, isopropanol, ethylene glycol, propylene glycol, methyl acetate, ethyl acetate, acetone, methyl ethyl ketone, 1,3-dioxane, 1,4-dioxane, N-methyl-2-pyrrolidone, tetrahydrofuran and dimethyl sulphoxide. Preferably, the solvent or solvent mixture will contain at least some water. Examples of water-containing solvent mixtures include water-methanol, water-ethanol, water-propylene glycol and water-ethylene glycol. Tertiary or higher systems may be employed, if desired. Most preferably, however, the solvent will be water.

The dye or dyes which are used in a given solution are selected so that the solution mimics the spectral response of whole blood at the seven wavelengths used in the Corning Model 2500 Cooximeter. If a single dye is used, the dye usually will be reddish-blue. If more than one dye is used, the first dye will typically be a red dye, with the addition of a yellow, pink, brown, black and/or blue dye as required. It will be apparent to those skilled in the art that there is an unavoidable degree of trial and error involved. Moreover, lot-to-lot variations are common with dyes and contribute to some extent to the need for reasonable experimentation.

In general, the concentration of each dye in a given solution will be dictated by the extinction coefficients of the dye at the wavelengths utilized by the instrument, as modified by the presence of other dyes and/or adjuvants. As used herein, the term "adjuvant" refers to a material which shifts the spectral response curve of a dye or mixture or dyes in solution. The shifting of the spectral response curve necessarily results in changes in the extinction coefficients of the dye or dye mixture for at least some wavelengths. As a general rule, dye concentrations may vary from 0.1 to 15 g/l.

Examples of suitable dyes include Atacryl yellow 4G, methyl violet, Atacryl blue GRLA 300%, Atacryl black NJ, Atacryl blue 4G, basic black TN, Atalan yellow NW, levafix brilliant yellow E-3G, resorcine brown Y, acid scarlet 2R, Atanyl yellow 3 GN, tartrazine, acid rubine 3GP, Atanyl red GN 200%, Atanyl yellow 4NGL, acid amaranth, acid rhodamine B (phloxine rhodamine), rhodamine B and phloxine B.

The solution of one or more dyes may optionally contain other components, such as adjuvants, viscosity adjusters, buffers, surfactants and preservatives. The use of an adjuvant is preferred. In addition, the viscosity of the dye solution is preferably adjusted to be similar to that of blood so that the dye solution flows through the instrument in the same manner as patient samples. In an especially preferred embodiment, the adjuvant also functions as a viscosity adjuster.

Examples of materials which may be employed as adjuvants and/or viscosity adjusters include poly-vinylpyrrolidones of varying molecular weights, propylene glycol, dextrans of varying molecular weights, polyethylene glycols of varying molecular weights, vegetable gums, ethylene glycol and 2-methoxyethanol.

When the solution is water based, such solution may contain buffers, such as phosphate buffers; zwitterionic buffers, examples of which include 2-(N-morpholino)ethane sulphonic acid (MES), 3-(N-morpholino)-propane sulphonic acid (MOPS), N-(2-acetamido)iminodiacetic acid (ADA), piperazine-N,N'-bis(2-ethane sulphonic acid) (PIPES), N-(2-acetamido)-2-aminoethane sulphonic acid (ACES), (2-aminoethyl)-trimethylammonium chloride hydrochloride (cholamine chloride), N,N-bis-(2-hydroxyethyl)-2-aminoethane sulphonic acid (BES), N-tris(hydroxymethyl)-methyl-2-aminoethane sulphonic acid (TES), N-2-hydroxyethylpiperazine-N'-2-ethane sulphonic acid (HEPES), N-(2-acetamido)glycine (acetamidoglycine), N-tris-(hydroxymethyl)methylglycine (tricine), N,N-bis(2-hydroxyethyl)-glycine (bicine), and glycylglycine.

Other components which may be employed in the dye solutions include surfactants and preservatives (including antimicrobial agents), as are well known in the art. The use of such other components is not known to be critical, provided only that such components do not interact with other components present in the solution in a manner which significantly adversely affects solution and/or component stability.

The present invention is further illustrated by the following Examples.

Example 1

The cooximetry quality control composition described contains 8.4 g/l of phloxine rhodamine (sulfacide brilliant pink 3B or acid red 52, C.I. 45100) and 60 g/l of a polyvinylpyrrolidone having a molecular weight of about 40,000 as adjuvant and viscosity adjuster. The procedure employed for determining the amount of dye required is described below.

To each of four 60-ml portions of deionized water was added 0.300 ± 0.005 g, 0.600 ± 0.005 g, 0.900 ± 0.005 g and 1.200 ± 0.005 g, respectively, of phloxide rhodamine. When the dye was dissolved, 6.0 g of the polyvinylpyrrolidone was added to each of the resulting solutions. Each solution then was diluted with deionized water to a final volume of 100.0 ml and mixed thoroughly.

A Corning Model 2500 Cooximeter was calibrated with samples of blood having known THb concentrations. Each of the above solutions then was run in triplicate on the Cooximeter. The apparent THb value determined by the instrument was plotted against concentration of dye. The resulting data were subjected to a linear regression analysis. Each point was within ± 0.1 g/dl THb of the calculated regression line. The concentration of dye required to give a THb value of about 18.7 was then calculated from the regression line. Each of the four solutions was then used as a sample to determine the other three hemoglobin species, namely, percent $O_2Hb$, percent COHb and percent HiHb.

From the regression line calculation described above, it was determined that about 8.4 g/l of dye would be required to give a THb value of $18.7 \pm 0.2$ g/dl. Accordingly, 8.40 g of dye was dissolved in about 500 ml of dionized water and stirred until the dye had dissolved. To the resulting solution was added 60 g of polyvinylvinylpyrrolidone. The mixture was stirred until the polyvinylpyrrolidone was completely dissolved. The resulting solution then was diluted to 1.0 l with dionized water. The final solution was filtered successively through 1.2 μm and 0.2 μm filters.

The solution had a viscosity of $2.5 \pm 0.2$ cps, $(0.0025 \pm 0.0002$ Pas$)$, as measured on a Brookfield Viscometer at 37°C. The spectral response of the solution at the wavelengths employed by the Corning Model 2500 Cooximeter, when measured in a 0.1 mm cell on a Gilford Model 2600 spectrophotometer (Gilford Instrument laboratories, Inc., Oberlin, Ohio, U.S.A.), was as follows:

| Wavelength | Absorbance |
| --- | --- |
| 521 nm | 0.6965 |
| 534.5 nm | 0.8054 |
| 547 nm | 0.8796 |
| 585 nm | 0.5494 |
| 595 nm | 0.1964 |
| 627 nm | 0.0105 |
| 660 nm | 0.0052 |

When run as a sample on a Corning Model 2500 Cooximeter, the solution gave the following results:

| | |
|---|---|
| THb | 18.7 g/dl |
| O$_2$Hb | 18.1% |
| COHb | 15.7% |
| HiHb | 40.7% |
| HHb | 25.6% |

Accelerated stability studies on the solution of Example 1 packaged in glass ampoules indicated that the composition is stable for at least one year at ambient termperature.

Example 2

Following the procedure of Example 1, a cooximetry quality control composition was prepared which consisted of the following components:

| | |
|---|---|
| acid rhodamine B | 2.48 g/l |
| levafix brilliant yellow E-3G | 13.0 g/l |
| phloxine B | 0.2 g/l |
| polyvinylpyrrolidone | 60.0 g/l |

The final solution had a viscosity of 2.5 $\pm$ 0.2 cps (0.0025 $\pm$ 0.0002 Pas) and gave the following spectral response:

| Wavelength | Absorbance |
|---|---|
| 521 nm | 0.2340 |
| 534.5 nm | 0.2981 |
| 547 m | 0.4157 |
| 585 nm | 0.2394 |
| 595 nm | 0.0981 |
| 627 nm | 0.0147 |
| 660 nm | 0.0135 |

When run as a sample on a Corning Model 2500 Cooximeter, the solution gave the following results:

| | |
|---|---|
| THb | 8.65 g/dl |
| O$_2$Hb | 10.3% |
| COHb | 18.1% |

| HiHb | 2.1% |
|------|------|
| HHb | 69.5% |

Example 3

Following the procedure of Example 1, a cooximetry quality control composition was prepared which consisted of the following components:

| Acid rhodamine B | 4.42 g/l |
|------------------|----------|
| levafix brilliant yellow E-3G | 12.75 g/l |
| phloxine B | 0.263 g/l |
| polyvinylpyrrolidone | 60.0 g/l |

The final solution had a viscosity of $2.5 \pm 0.2$ cps ($0.0025 \pm 0.0002$ Pas) and gave the following spectral response:

| Wavelength | Absorbance |
|------------|------------|
| 521 nm | 0.3943 |
| 534.5 nm | 0.4925 |
| 547 nm | 0.6563 |
| 585 nm | 0.3960 |
| 595 nm | 0.1544 |
| 627 nm | 0.0086 |
| 660 nm | 0.0061 |

When run as a sample on a Corning Model 2500 Cooximeter, the solution gave the following results:

| THb | 15.2 g/dl |
|-----|-----------|
| $O_2Hb$ | 18.6% |
| COHb | 7.4% |
| HiHb | 15.1% |
| HHb | 58.8% |

Example 4

Following the procedure of Example 1, a cooximetry quality control composition was prepared which consisted of the following components:

| acid rhodamine B | 4.44 g/l |
|------------------|----------|

| | | |
|---|---|---|
| Atanyl yellow 4 NGL | 4.5 g/l | |
| phloxine B | 0.25 g/l | |
| polyvinylpyrrolidone | 60.0 g/l | |

The final solution had a viscosity of 2.5 ± 0.2 cps (0.0025 ± 0.0002 Pas) and gave the following spectral response:

| Wavelength | Absorbance |
|---|---|
| 521 nm | 0.5373 |
| 534.5 nm | 0.6762 |
| 547 nm | 0.8992 |
| 585 nm | 0.5510 |
| 595 nm | 0.2197 |
| 627 nm | 0.0134 |
| 660 nm | 0.0102 |

When run as a sample on a Corning Model 2500 Cooximeter, the solution gave the following results:

| | |
|---|---|
| THb | 14.4 g/dl |
| $O_2Hb$ | 19.9% |
| COHb | 5.7% |
| HiHb | 16.0% |
| HHb | 58.4% |

Example 5

Following the procedure of Example 1, a cooximetry quality control composition was prepared which consisted of the following components:

| | |
|---|---|
| phloxine rhodamine | 3.75 g/l |
| Atanyl yellow 4NGL | 0.71 g/l |
| polyvinylpyrrolidone | 60.0 g/l |

The final solution had a viscosity of 2.5 ± 0.2 cps (0.0025 ± 0.0002 Pas) and gave the following spectral response:

| Wavelength | Absorbance |
|---|---|
| 521 nm | 0.3943 |
| 534.5 nm | 0.4925 |
| 547 nm | 0.6563 |
| 585 nm | 0.3960 |
| 595 nm | 0.1544 |
| 627 nm | 0.0086 |
| 660 nm | 0.0061 |

When run as a sample on a Corning Model 2500 Cooximeter, the solution gave the following results:

| | |
|---|---|
| THb | 8.74 g/dl |
| $O_2Hb$ | 32.3% |
| COHb | 0.0% |
| HiHb | 35.2% |
| HHb | 32.5% |

CLAIMS:

1.    A cooximetry quality control composition characterised in that it is free from blood-derived components
and comprises a solution of one or more dyes, which
solution mimics the spectral response of whole blood at
a plurality of wavelengths in the visible region of the
spectrum.

2.    A composition as claimed in claim 1 wherein the
solution is a substantially aqueous solution.

3.    A composition as claimed in claim 1 or claim 2
wherein the solution contains an adjuvant.

4.    A composition as claimed in any of claims 1 to 3
wherein the solution contains a viscosity adjuster.

5.    A composition as claimed in any of claims 1 to 4
wherein the solution contains a component which functions
both as an adjuvant and as a viscosity adjuster.

6.    A composition as claimed in claim 5 wherein the
component is a polyvinylpyrrolidone.

7.    A composition as claimed in any of claims 1 to 6
wherein the solution contains a single dye.

8.    A composition as claimed in any of claims 1 to 6
wherein the solution contains two or more dyes.

9.    A composition as claimed in any of claims 1 to 8
wherein the dye(s) is/are selected from acid rhodamine B,
phloxine rhodamine, phloxine B, levafix brilliant yellow
E-3G and Antanyl yellow 4NGL.

10.   The use of a cooximetry quality control composition
as claimed in any of claims 1 to 9.

## EUROPEAN SEARCH REPORT

European Patent Office

Application number

EP 84304969.3

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.4) |
| A | EP - A2 - 0 031 786 (A.L. LOUDER-BACK et al.)<br>* Claims 1-8 * | 1-5,7, 8 | G 01 N 33/52<br>G 01 N 33/72<br>G 01 N 33/96 |
| D,A | US - A - 4 001 142 (J.E. TURNER)<br>* Claims 1-6 * | 1-5,7, 8 | |

**TECHNICAL FIELDS SEARCHED (Int. Cl.4)**

G 01 N 33/00

The present search report has been drawn up for all claims

| Place of search | Date of completion of the search | Examiner |
|---|---|---|
| VIENNA | 24-10-1984 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82